# EUROPEAN PATENT APPLICATION

(11) **EP 0 779 060 A2**
(43) Date of publication of application: **18.06.1997**
(21) Application number: 96116345.8
(22) Date of filing: 11.10.1996
(51) Int. Cl.: A61B 17/39

(54) **Grounded electrode surgical implement and system**

(30) Priority: 11.12.1995 US 570640
(71) Applicant: MEGADYNE MEDICAL PRODUCTS, INC., Draper, UT 84020 (US)
(72) Inventor: Nettekoven, William S., Sandy, Utah 84092 (US)
(74) Representative: Wagner, Karl H., Dipl.-Ing.

(57) **Abstract**

An improved electrosurgical instrument having a first primary covering of insulation covering the surface except in a working location and a location provided for connection thereto of a source of electrocautery high frequency voltage. Overlying the first covering of insulation is a conductive covering adapted for connection to a source of ground potential for safely diverting electrical potential that may otherwise develop because of undesired capacitive coupling or that may escape through the primary insulation in the event of failure thereof. Alternatively, or in addition thereto, provision is made for connection to impedance measuring circuitry for monitoring the condition of the primary insulation and warning of deterioration of such insulation. Overlying the conductive covering, there is provided a second coating of insulation for protecting the conductive covering and enhancing safety in use of the implement.

## Description

This invention relates to medical instruments that are adapted for use with electrosurgical techniques and more particularly to such instruments for use in electrocautery.

### BACKGROUND OF THE INVENTION

As is known to those skilled in the art, there is a high level of concern in medical circles for patient and surgeon safety in performing medical procedures. As surgical knowledge and techniques have progressed, there has been a corresponding trend toward size reduction of surgical incisions and invasive instruments, thus decreasing patient trauma and contributing to rapidity of patient recovery. This has led to the practice of laparoscopic and other surgical procedures using small medical electrodes. The practice of electrosurgery and more particularly electrocautery has presented many advantages. However, its use has involved the application of high frequency electrical potentials which are hazardous to patients and/or attending medical personnel unless they are confined to the intended point of application. Thus, the conductive path leading from the high frequency electrical source to the point of application typically is insulated to prevent escape and possible burning of the patient or attending medical personnel.

As is known to those skilled in the art, a variety of electrosurgical techniques and implements have heretofore been proposed, illustrative of which are those described in United States Patent 1,916,722 granted to F. M. Ende July 4, 1933; German Patent 2,404,764 granted to Bernard Weissman, et al. 19 September, 1974; PCT International Application US82/0084 filed by William S. Walker 25 January, 1982; PCT International Application US 91/05520 filed by Edwin Langberg 2 August, 1991; and United States Patent Application Serial Number 08/342,215 filed November 18, 1994. According to their proposals, multi-element implements have included reusable bodies with removable and disposable or reusable tips or electrodes. The main bodies or holders may be sterilized if needed and re-used, and the tips, blades or electrodes either discarded after one use or sterilized and re-used. However, use and cleaning of such devices increases vulnerability to insulation failure, thus heightening the need for enhancing care in control of electrosurgical voltages.

In addition to the foregoing, it is known that in certain instances, undesired capacitive coupling may occur between an intentionally energized device such as a surgical electrode and adjacent elements or substances such as a trocar or tissue especially when a surgical electrode is activated and its tip or working member is not in contact with tissue, thus leaving no path for capacitive charge to dissipate. If the potential between the electrode and the tissue or conductive element becomes large enough, it can lead to insulation failure and arcing, resulting in damage to surrounding tissue. Accordingly, there has been a continuing need to develop improved techniques for enhancing safety and protection.

### BRIEF SUMMARY OF THE INVENTION

The foregoing improved techniques are provided in an improved medical implement according to the invention hereof which includes a conducting working member to conduct the electrosurgical electrical potential to its intended point of application to a patient, a first insulating coating on the exterior of the working member other than at its intended location of application to a patient, an electrically conducting covering overlying the first insulating coating, an electrical connection to the electrically conducting covering for applying an electrical grounding potential thereto, and a second insulating coating overlying the electrically conducting covering for providing a second and exterior insulating covering for the implement.

### OBJECTS AND FEATURES OF THE INVENTION

It is one general object of the invention to improve electrosurgical implements.

It is another object of the invention to improve safety in use of such implements.

It is another object of the invention to reduce vulnerability of electrosurgical implements to insulation failure.

It is yet another object of the invention to detect incipient insulation deterioration.

It is still one other object of the invention to protect against undesired capacitive coupling build-up of potentials between an electrosurgical implement and adjacent elements or substances.

Accordingly, in accordance with one feature of the invention, a first covering of insulation is provided on an electrosurgical implement to cover the entire surface thereof excepting the working region of the implement and a region provided for connecting a source of energizing potential thereto; and a grounding sheath is provided over the first covering of insulation, thus providing protection against undesired capacitive coupling build-up of potentials as well as for a relatively harmless dissipation of electrical potential in the event of some capacitive coupling or failure of the first covering of insulation.

In accordance with another feature of the invention, a second covering of insulation is disposed over the surface of the grounding sheath, thus providing an additional protective layer to prevent untoward electrical contact with either patient or implement user.

In accordance with still another feature of the invention, provision is made so that the grounding sheath may be connected either directly to the patient ground or alternatively by electrical leakage-sensing circuits; in the latter case thereby to sense incipient insulation damage or impending insulation failure and further improve safety.

These and other objects and features of the invention will be apparent from the following description, by way of example of a preferred embodiment, with reference to the drawing.

### BRIEF DESCRIPTION OF THE DRAWING

Figure 1 is a view depicting an electrosurgical implement constructed according to the invention; and
Figure 2 is a diagram illustrating the implement of Figure 1 connected to insulation condition-sensing circuits.

### DESCRIPTION OF PREFERRED EMBODIMENTS

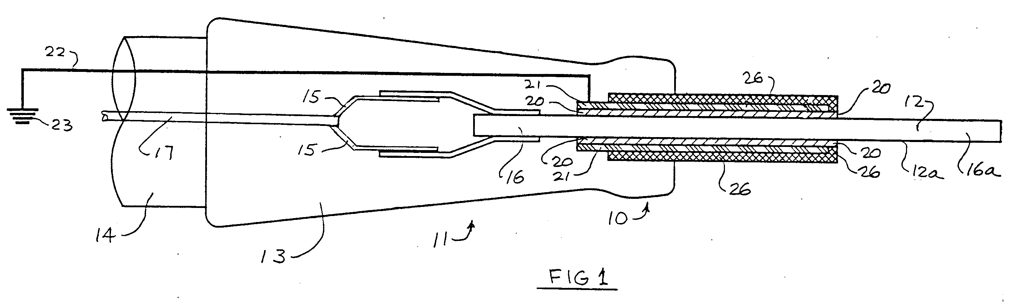

Now turning to the drawing, and more particularly Figure 1 thereof, it will seen to illustrate a composite electrosurgical implement generally shown at 10. Implement 10 may be a single device (as shown) or it maybe divisible into two or more sections as described in the patents/application to which reference is made above. At the left side of the figure there is shown a conventional electrosurgical pencil 11 in which there is mounted electrosurgical electrode 12.

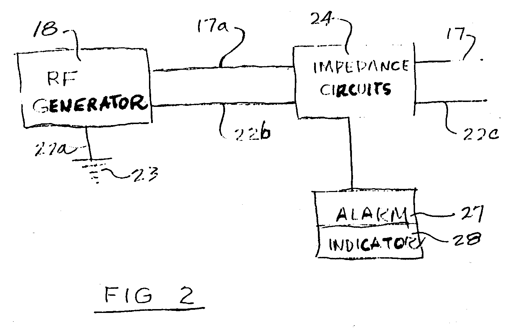

Pencil 11 comprises conventional handle portion 13 with a conventional extension 14 and internal connector 15 for making electrical contact between exposed electrode shank portion 16 of electrode 12 and cable 17 which leads to source 18 of electrosurgical voltage (Figure 2).

Disposed on surface 12a of electrode 12 (and covering it entirely except for its proximal and distal ends 16 and 16a), there is a first (primary) covering of insulation 20 which is affixed to surface 12a by conventional techniques. Overlying primary insulation 20 is an electrically conducting layer 21 that preferably is a thin continuous metallic sheet, although it could be provided as a braid or other form. Connected to conducting layer 21 is an electrical connector such as a conventional cable 22 that leads either directly to ground 23 as depicted in Figure 1 or to impedance-sensing circuits 24 as illustrated in Figure 2. Overlying conducting layer 21 is a second insulating layer 26 that overlies all or most of conducting layer 21. The purpose of layer 26 is to provide covering for conducting layer 21 to protect it from physical damage as well as to provide additional protection to user personnel from electrosurgical voltage if for any reason (such as insulating failure, physical damage, capacitive coupling or the like) it should escape past conducting covering 21.

As is known to those skilled in the art, when practicing certain types of surgery, a portion of an electrosurgical instrument is often obscured from view of the surgeon, thus heightening the importance of having the insulating and conducting layers extend for the entire length of an electrode other than the intentionally exposed working end or tip. However, it should be understood that in certain instances, principles hereof may be employed under circumstances in which a part of one or more of the coverings may extend over less than the entire length of the electrode.

Now turning to Figure 2, it will be seen to be a diagram illustrating the implement of Figure 1 connected to insulation sensing circuits. There, are seen schematically depicted, the aforementioned connections from the electrosurgical pencil of Figure 1 together with the grounding and impedance sensing circuits for monitoring the condition of the primary insulating layer 20.

Although a connection may be made from conducting covering 21 via conductor 22 directly to ground as shown in Figure 1, in an alternative embodiment, provision is made for inclusion of the impedance sensing and alarm circuits of Figure 2. Thus, r.f. activating voltage is extended from r.f. generator 18 via cable 17a through impedance monitoring circuits 24 and thence via r.f.cable 17 to electrosurgical implement 10 (Figure 1); and ground potential is applied to conducting covering 21 from ground 23 via conductor 22a, through r.f. generator 18, conductor 22b, impedance sensing circuits 24 and conductor 22c to electrosurgical implement 10.

R.f. generator 25 may be any of a number of such generators known to those skilled in the art; and impedance monitoring circuits may be simple conventional impedance measuring circuits that either measure the impedance itself directly or monitor the power factor of the applied r.f.voltage when the implement is not in contact with a patient. The precise level of impedance that marks a dangerous threshold indicative of present or impending insulation failure will vary depending upon the type of insulation employed, its thickness, and the area in which it contacts the outer surface 12a of electrode 12; and the principles upon which it is computed are well known to those skilled in the art.

Further reference to Figure 2 reveals the presence of optional alarm 27 and visual indicator 28. These may be connected to impedance monitoring circuits 24 and be activated by an electrical indicia indicative of lowered impedance in any of a variety of ways known to those skilled in the art. Thus, the alarm may be audible such as by sounding a buzzer, tone, or other sound; and/or the alarm may be communicated by a visual indication such as a blinking red light.

It will now be evident that there has been described above an electrosurgical implement and monitor which provide an increased margin of safety for both patient and physician.

Although the inventions hereof have been described by way of preferred embodiments, it will be evident that other adaptations and modifications may be employed without departing from the spirit and scope thereof. For example, an additional conducting covering could be disposed over the outer insulating covering.

The terms and expressions employed herein have been used as terms of description and not of limitation; and thus, there is no intent of excluding equivalents, but on the contrary it is intended to cover any and all equivalents that may be employed without departing from the spirit and scope of the invention.

It should be noted that the objects and advantages of the invention may be attained by means of any compatible combination(s) particularly pointed out in the items of the following summary of the invention and the appended claims.

### SUMMARY OF THE INVENTION

1. An electrosurgical implement comprising:
   (a) a metallic shaft having a distal end and an electrosurgical working surface at said distal end, a proximal end including means at said proximal end for connecting electrocautery voltage to said metallic shaft, and a principal surface extending between said working surface and said proximal end
   (b) a first covering of insulation overlying said principal surface;
   (c) a conducting layer overlying said first covering of insulation; and
   (d) means for interconnecting said conducting layer with a source of electrical ground.
2. An electrosurgical implement
   further including a second insulating layer overlying said conducting layer.
3. An electrosurgical implement
   wherein said conducting layer partially overlies said first covering of insulation.
4. An elecrosurgical implement
   wherein said conducting layer essentially entirely overlies said first covering of insulation.
5. An electrosurgical implement
   further including means for monitoring electrical impedance between said conducting layer and said metallic shaft.
6. An electrosurgical implement
   wherein said means for monitoring includes means for producing an alarm indicia when impedance between said metallic shaft and said conducting layer decreases to a predetermined level.
7. In combination, an electrosurgical implement and manipulating means having a handle mounted to said implement for manipulating said implement.
8. An electrosurgical implement
   further including means for monitoring electrical impedance between said conducting layer and said metallic shaft.
9. In combination, an electrosurgical implement and manipulating means having a handle mounted to said implement for manipulating said implement.
10. An electrosurgical implement
   wherein said conducting layer partially overlies said first covering of insulation.
11. An electrosurgical implement
   further including means for monitoring electrical impedance between said conducting layer and said metallic shaft.
12. An electrosurgical implement
   wherein said conducting layer essentially entirely overlies said first covering of insulation.
13. An electrosurgical implement according to Claim 12 further including means for monitoring electrical impedance between said conducting layer and said metallic shaft.
14. An electrosurgical implement
   further including means for monitoring electrical impedance between said conducting layer and said metallic shaft.
15. An electrosurgical implement
   wherein said means for monitoring includes means for producing an alarm indicia when impedance between said metallic shaft and said conducting layer decreases to a predetermined level.
16. In combination, an electrosurgical implement and manipulating means having a handle mounted to said implement for manipulating said implement.

## Claims

1. An electrosurgical implement comprising:
(a) a metallic shaft having a distal end and an electrosurgical working surface at said distal end, a proximal end including means at said proximal end for connecting electrocautery voltage to said metallic shaft, and a principal surface extending between said working surface and said proximal end
(b) a first covering of insulation overlying said principal surface;
(c) a conducting layer overlying said first covering of insulation; and
(d) means for interconnecting said conducting layer with a source of electrical ground.

2. An electrosurgical implement according to Claim 1 further including a second insulating layer overlying said conducting layer.

3. An electrosurgical implement according to Claim 2 wherein said conducting layer partially overlies said first covering of insulation.

4. An elecrosurgical implement according to Claim 2 wherein said conducting layer essentially entirely overlies said first covering of insulation.

5. In combination, an electrosurgical implement according to on or more of the proceeding Claims and manipulating means having a handle mounted to said implement for manipulating said implement.

6. An electrosurgical implement according to one or more of the proceeding Claims wherein said conducting layer partially overlies said first covering of insulation.

7. An electrosurgical implement according to one or more of the proceeding Claims further including means for monitoring electrical impedance between said conducting layer and said metallic shaft.

8. An electrosurgical implement according to one or more of the proceeding Claims wherein said means for monitoring includes means for producing an alarm indicia when impedance between said metallic shaft and said conducting layer decreases to a predetermined level.

9. In combination, an electrosurgical implement according to one or more of the proceeding Claims and manipulating means having a handle mounted to said implement for manipulating said implement.

10. An electrosurgical implement comprising:
a metallic shaft having a distal end and an electrosurgical working surface at said distal end, a proximal end including means at said proximal end for connecting electrocautery voltage to said metallic shaft, and a principal surface extending between said working surface and said proximal end
